# EUROPEAN PATENT APPLICATION

(11) **EP 1 790 282 A2**
(43) Date of publication of application: **30.05.2007**
(21) Application number: 06256050.3
(22) Date of filing: 27.11.2006
(51) Int. Cl.: A61B 5/00, A61B 5/0285

(54) **Method of measuring blood circulation velocity by controlling breath**

(30) Priority: 28.11.2005 CN 200510126928
(71) Applicant: Zen-u Biotechnology Co., Ltd, Sindian City, Taipei 231 (TW)
(72) Inventor: Lu, Hsueh-Kuan, Taichung City 400, Taiwan (TW); Lu, Chih-Yi, Chiayi County 606 (TW)
(74) Representative: Prentice, Raymond Roy

(57) **Abstract**

A method of measuring blood circulation velocity by controlling a person's breath includes the steps of keeping the person in a first breathing status with a blood oxygen saturation analytical instrument; setting an initial time point and starting to record a blood oxygen saturation value per predict time interval; requesting the person to change into a second breathing status at a first time point; requesting the person to change into a third breathing status at a second time point; stopping recording the blood oxygen saturation value at a terminal time point; setting a reference time point according to the blood oxygen saturation value which has a variation according to the records; obtaining the person's blood circulation velocity, which is proportional to the reciprocal of the difference of the reference time point and the first time point.

## Description

### BACKGROUND

The present invention relates to methods of measuring blood circulation velocity for obtaining a person's blood circulation velocity, and more particularly to a method of measuring blood circulation velocity by controlling breath of the person to be measured.

In recent years, when people's health is getting worse, they start valuing the related subject of heath. People look for treat by food, physics treatment or medicine cure method and etc., trying to improve their healthy condition. However, the health has no absolute quantity index, the people usually regard the quality of their blood circulation condition as one kind of reference index. In other words, when blood circulation condition is not well, the healthy condition may not be well either; vice versa, the healthy condition might be also well.

However, how to exactly judge the blood circulation condition of a person is well or not? One kind of conventional method is by injecting photosensitive matter into the person's blood firstly, then utilizing photosensitive photograph or taking photo to observe blood circulation of the person to be measured. Nevertheless, this way can only acquire blood circulation condition to be measured once, but can not repeat to obtain blood circulation velocity quantity.

In addition, another conventional method is using a blood current velocity measurement manufactured according to the principle of the Dopplor effect. The theory of this method is that the flowing blood may induce the anti-diffusing photons to generate Dopplor frequency shift, and the quantity of the Dopplor frequency shift is proportional to the blood current velocity; so using an Optical Doppler Tomography (ODT) to scan the Dopplor frequency shift would obtain the blood current velocity. However, this method is not easy to carry out, it is because that although each Dopplor frequency shift is certain corresponding to a particular object, the fluxion of blood is continuous, therefore; the measurement value isn't accurate and the emersion is lower. Moreover, the scanning instrument, such as Optical Doppler Tomography, is expensive, so this conventional method isn't widespread.

Accordingly, what is needed is a method of measuring blood circulation velocity that can overcome the above-described deficiencies.

### BRIEF SUMMARY

The present invention provides a method of measuring blood circulation velocity, and the method is realized by controlling one person's breath and using a blood oxygen saturation analytical instrument to rapidly measuring and obtaining blood circulation velocity of the person.

The method of measuring blood circulation velocity by controlling a person's breath includes the steps of: keeping the person in a first breathing status with a blood oxygen saturation analytical instrument; setting an initial time point and starting to record a blood oxygen saturation value per predict time interval; requesting the person to change into a second breathing status at a first time point; requesting the person to change into a third breathing status at a second time point; stopping recording the blood oxygen saturation value at a terminal time point; setting a reference time point according to the blood oxygen saturation value which has a variation according to the records; obtaining the person's blood circulation velocity, which is proportional to the reciprocal of the difference of the reference time point and the first time point.

According to the above-mentioned method, the first breathing status is a normal breathing status of the person to be measured; and the second breathing status is a short breathing status or a deep breathing status of the person to be measured.

According to the above-mentioned method, if the second breathing status is the short breathing status, the person should empty the air inside the body before or at the same time of acting the short breathing status.

According to the above-mentioned method, the third breathing status is the same of the first breathing status.

According to the above-mentioned method, the blood circulation velocity of the person to be measured is obtained by using a reference distance dividing the difference of the reference time point and the first time point, wherein the reference distance is proportional to the height of the person to be measured, and then which may be corrected according to the height of the person to be measured.

According to the above-mentioned method, the first time point is at about 20th second, the second time point is at about 40th second, and the terminal time point is at about 90th second.

According to the above-mentioned method, the blood oxygen saturation analytical instrument is not an intrusive type blood oxygen saturation analytical instrument.

According to the above-mentioned method, if the blood oxygen saturation value at the first time point is different to that at the initial time point, then make the record of the blood oxygen saturation value invalid.

### BRIEF DESCRIPTION OF THE DRAWINGS

These and other features and advantages of the various embodiments disclosed herein will be better understood with respect to the following description and drawings, in which like numbers refer to like parts throughout, and in which:

Figure 1 is a block diagram of a method of measuring blood circulation velocity according to an exemplary embodiment of the present invention;

Figure 2 is a curve diagram of blood oxygen saturation values recorded according to a plurality of time points by using the method of figure 1, in which the person to be measured acting in a short breathing status; and

Figure 3 is another curve diagram of blood oxygen saturation values recorded according to a plurality of time points by using the method of figure 1, in which the person to be measured acting in a deep breathing status.

### DETAILED DESCRIPTION

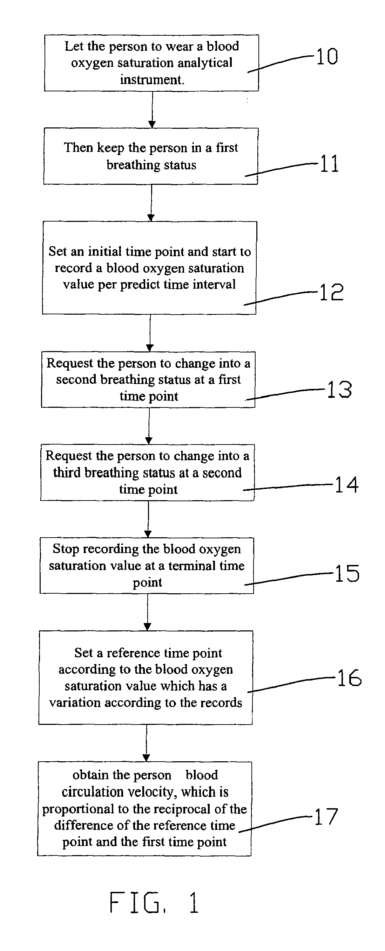

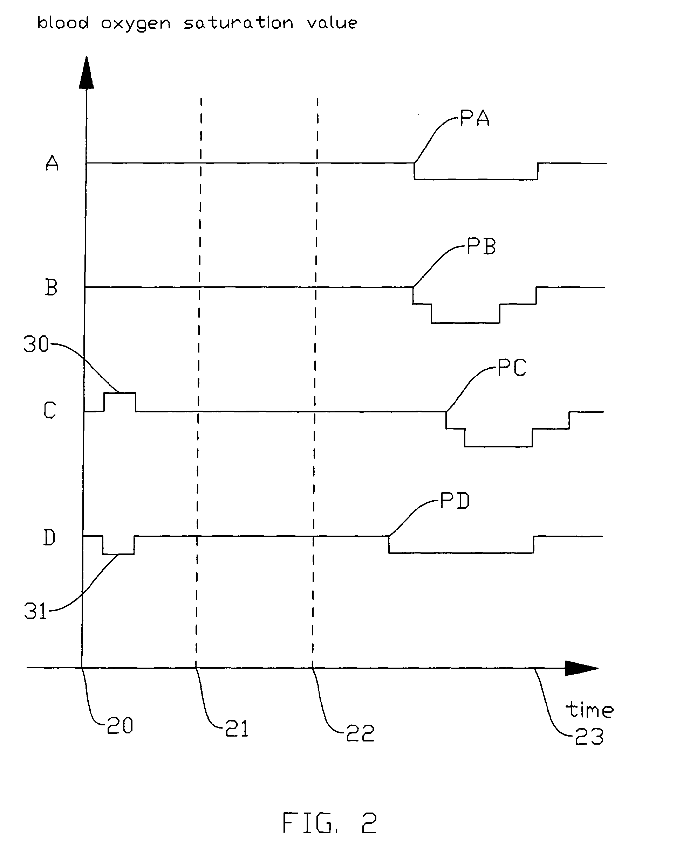

Referring to figures 1 and 2, figure 1 is a block diagram of a method of measuring blood circulation velocity according to an exemplary embodiment of the present invention; and figure shows four sets of curve of blood oxygen saturation values recorded according to a plurality time points by using the method of figure 1. The detail steps of the method are explained as follows.

Firstly, according to step 10 of figure 1, let the person to wear a blood oxygen saturation analytical instrument. The blood oxygen saturation analytical instrument is used to detect the blood oxygen saturation value of the person to be measured, which may be an intrusive type blood oxygen saturation instrument, such as OXY100C. This kind of the instrument would not make the person to induce destructive injury, and it only needs to tightly clip the finger tip of the person for measuring the blood oxygen saturation values thereof. The method should not be limited with this limitation, preferably, an automatic record device (hardware or software) may be equipped for recording the blood oxygen saturation values of the person to be measured corresponding to time points to form the curve of the blood oxygen saturation values of figure 2. Then keep the person in a first breathing status, according to step 11 of figure 1. Preferably, the person to be measured maintains a normal breathing status under the condition without interference of outside matters, such as quietly lying or sitting down in silence, which is easier to make the person to maintain a normal breathing status. Under this condition, the breath of the person is natural and smooth, and the curve of the blood oxygen saturation values obtained by the blood oxygen saturation instrument should approximate to a horizontal straight line corresponding to the x-coordinate of time.

After that, set an initial time point 20 and start to record a blood oxygen saturation value per predict time interval according to step 12 of figure 1. Preferably, it does not to make the person to know the initial time point so as to avoid disturbing the record of the blood oxygen saturation values. Because the person is in the first breathing status, i.e. the normal breathing status, the curve of the blood oxygen saturation values is approximately to a horizontal straight line corresponding to the x-coordinate of time, referring to curve A and B of figure 2.

Then, request the person to change into a second breathing status at a first time point 21, such as at about 20th second, according to step 13 of figure 1. In this embodiment, the second breathing status is to make the person to be measured in a short breathing status. Preferably, the person should empty the air inside the body before acting the short breathing status at the time point 21 so as to improve the veracity of the measuring result. After that, request the person to change into a third breathing status at a second time point 22, such as at 40th second, according to step 14 of figure 1. It only needs to make the third breathing status different to the second breathing status, preferably, the third breathing status is the same as the first breathing status, that is, make the person come back to normal breathing status. The method may be realized only request that the third breathing status different to the second breathing status. Then, stop recording the blood oxygen saturation value at a terminal time point 23, such as at about 90th second, according to step 15 of figure 1. The terminal time point 23 may be different corresponding to different detecting points, such as finger tip, toe tip, or the like. Properly setting a detecting point, it can obtain a plurality of blood oxygen saturation value samples to form a curve, such as curve A or curve B of figure 2. The first time point 21 may not be limited to be at about 20th second, the second time point 22 may not be limited to be at about 40th second, and the terminal time point 23 may not be limited to be at about 90th second.

After that, set a reference time point according to the blood oxygen saturation value which has a variation according to the records, according to the step 16 of figure 1. Such as the PA point of figure 2, it denotes that the blood oxygen saturation value is decreased at PA point, and then the time point of the PA point is taken as the reference time point. For similar reasons, PB point also denotes that the blood oxygen saturation value is decreased thereat, which may also be taken as the reference time point. Then it can obtain the person's blood circulation velocity, which is proportional to the reciprocal of the difference of the reference time point and the first time point, according to the step 17 of figure 1.

According to the method above-mentioned, the theory of this method is that when the person to be measured acts in a short breathing status, the blood oxygen quantity of the lung bubble of the person is decreased immediately. But the blood in the body of each of the person has a different velocity, the reducing blood oxygen saturation value due to the decreasing of the blood oxygen quantity of the lung bubble would response at the detecting points, such as finger tip or the like after a certain time period, to be obtained by the blood oxygen saturation analytical instrument. The time period of this process is the reference time point minus the first time point, and the reciprocal of this time period is proportional to the blood circulation velocity of the person to be measured. It is hard to exactly measure the flowing distance of the blood between the lung bubble and the finger tip of the person to be measured. However, the flowing distance of a certain person is the same, so the emersion of this method of measuring the blood circulation velocity reference value is very high, which can easily and exactly be applied.

Moreover, though it is hard to exactly measure the flowing distance of the blood between the lung bubble and the finger tip of the person to be measured, the flowing distance of the blood of the person is proportional to the height thereof. Then it may set up a reference distance table, in which the reference distance is proportional to the height of the person to be measured. So the blood circulation velocity value of the person can be obtained by using the reference distance dividing the time of the difference of the reference time point and the first time point. Such that, the value of the blood circulation velocity measured by the method according to the present invention may be a valid reference value. On the other hand, it can directly set up a curve diagram of the relative variables relative to blood circulation velocity, and then the person can obtain the blood circulation velocity by comparing the above-mentioned reference value of the blood circulation velocity to the curve diagram. The relative variables may be relative to sex, age, height, body temperature, blood pressure, red corpuscle, RBC distribution width, deformability of erythrocytes, hematocrit, blood viscosity, basal metabolism, blood vessel diameter etc. It also can directly set up a blood velocity normal distribution table of a healthy person, and then the person to be measured can conveniently look up the healthy index of the blood circulation velocity in the blood velocity normal distribution table.

For improving the veracity of this method, preferably, if the blood oxygen saturation value at the first time point 21 is different to that at the initial time point 20, referring to the variation points 30 and 31 of figure 2, it denotes that the person to be measured doest not act a normal breathing status, the blood circulation velocity measured under this condition is not proper, even if it can obtain the points PC and PD afterwards, it should make the record of the blood oxygen saturation values invalidly. This step can improve the emersion of the method according to the present invention.

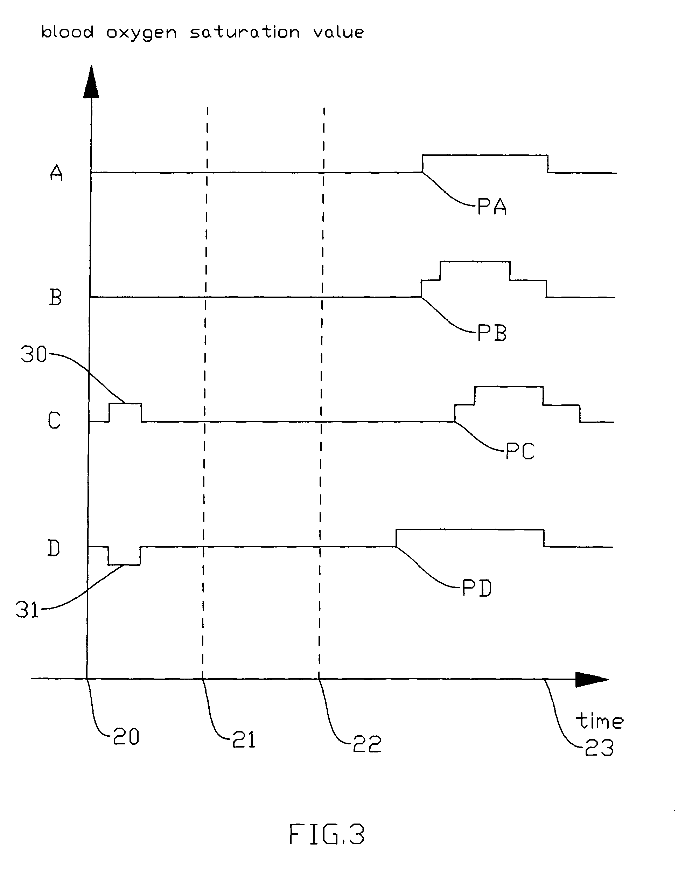

Furthermore, the method is not limited to make the person to act a short breathing status between the first and second time points 21 and 22, but the person to be measured can act a deep breathing status between the first and second time points 21 and 22. Referring to figure 3, because the deep breathing status of the person may increase the blood oxygen quantity between the lung bubble and the blood vessel, which directly increases the blood oxygen saturation value of the person to be measured, so the points PA and PB of the curves A and B denote that the blood oxygen saturation value of the person are increased. Then the time points corresponding to the points PA and PB can be set as reference time points for similar reasons described above to obtain the blood circulation velocity.

According to above-mentioned, the method of measuring the blood circulation velocity according to the present invention only use a blood oxygen saturation analytical instrument, which has lower cost and high emersion.

The above description is given by way of example, and not limitation. Given the above disclosure, one skilled in the art could devise variations that are within the scope and spirit of the invention disclosed herein, including configurations ways of the recessed portions and materials and/or designs of the attaching structures. Further, the various features of the embodiments disclosed herein can be used alone, or in varying combinations with each other and are not intended to be limited to the specific combination described herein. Thus, the scope of the claims is not to be limited by the illustrated embodiments.

## Claims

1. A method of measuring blood circulation velocity by controlling a person's breath, comprising the steps of:
keeping the person in a first breathing status with a blood oxygen saturation analytical instrument;
setting an initial time point and starting to record a blood oxygen saturation value per predict time interval;
requesting the person to change into a second breathing status at a first time point;
requesting the person to change into a third breathing status at a second time point;
stopping recording the blood oxygen saturation value at a terminal time point;
setting a reference time point according to the blood oxygen saturation value which has a variation according to the records; and
obtaining the person's blood circulation velocity, which is proportional to a reciprocal of a difference of the reference time point and the first time point.

2. The method as claimed in claim 1, wherein the first breathing status is a normal breathing status of the person to be measured.

3. The method as claimed in claim 1, wherein the second breathing status of the person to be measured is a short breathing status.

4. The method as claimed in claim 3, wherein the person empty air inside the body before or at the same time of acting the short breathing status.

5. The method as claimed in claim 1, wherein the second breathing status of the person to be measured is a deep breathing status.

6. The method as claimed in claim 1, wherein the third breathing status is the same of the first breathing status.

7. The method as claimed in claim 1, wherein the blood circulation velocity of the person to be measured is obtained by using a reference distance dividing the difference of the reference time point and the first time point.

8. The method as claimed in claim 7, wherein the reference distance is proportional to a height of the person to be measured.

9. The method as claimed in claim 1, wherein the person's blood circulation velocity is obtained by comparing the reciprocal to a normal distribution table.

10. The method as claimed in claim 1, wherein the record of the blood oxygen saturation value is invalid if the blood oxygen saturation value at the first time point is different to that at the initial time point.
